# EUROPEAN PATENT APPLICATION

(11) **EP 0 625 344 A2**
(43) Date of publication of application: **23.11.1994**
(21) Application number: 94250096.8
(22) Date of filing: 15.04.1994
(51) Int. Cl.: A61F 9/02, A41D 13/00

(54) **Integrated mask and goggle structure**

(30) Priority: 20.04.1993 JP 20410/93; 15.06.1993 JP 32124/93
(71) Applicant: Takehara, Tadahiro, Osaka (JP)
(72) Inventor: Takehara, Tadahiro, Osaka (JP); Ando, Saburo, Tokyo (JP)
(74) Representative: Wablat, Wolfgang, Dr.Dr.

(57) **Abstract**

An improved and new integral mask and goggle structure adapted to be mounted on the face of a wearer and protect the face of the wearer agaist industrial hazards or the like is provided. The structure carries a mask (1) adapted to surround the mouth and the nose of a wearer, and a goggle (20) adapted to surround the eyes and detachably mounted to the mask (1). The structure further carries a sliding means for sliding the goggle on the mask between its operative and withdrawn position.

## Description

### BACKGROUND OF THE INVENTION

### Field of the invention

This invention relates to an integrated mask and goggle structure such as may be used for welders for protecting the face, respiratory organs system, or portions from industrial hazards, such as sparks, poisonous gases and dusts, and used for a surgeon for protecting the face during a surgical operation.

### Description of the Prior Art

There have been commonly used devices to protect the face, respiratory organs of a wearer from industrial hazards in the forms of a mask and a goggle without a means for joining to each other, which requires individually wearing thereof. Accordingly, it was time consuming and cumbersome to wear and remove such devices.

In addition, another disadvantage resides in the fact that a clearance between the mask and the goggle may allow the industrial hazards to enter the face therethrough. Consequently, the devices of these types may not fully protect the face of the wearer.

### SUMMARY OF THE INVENTION

It is therefore an object of the present invention to provide a new and improved integrated mask and goggle structure which can be readily and instantly mounted to protect the face of a wearer, while securely preventing industrial hazards from entering the face of the wearer therethrough.

Accordingly there is provided an integrated mask and goggle structure of the present invention which comprises a mask adapted to surround the mouth and the nose of a wearer, and a goggle adapted to surround the eyes and detachably mounted to the mask.

The integrated mask and goggle structure of the present invention further comprises a mounting member adapted to be fitted to a lower portion of the goggle, and a mounting means provided on the mounting member so as to detachably mount the mask thereto.

An integrated mask and goggle structure of the present invention further comprises a mask adapted to surround the mouth and the nose of a wearer, a goggle adapted to surround the eyes, and a mounting means disposed between the mask and the goggle such that the goggle is detachably mounted to the mask.

In another aspect, the present invention features an integrated mask and goggle structure which enables the goggle to move between its operative position and its withdrawn position on the mask. Accordingly to this aspect of the present invention, between the mask and the goggle is provided a means for slidably mounting the goggle on the mask. This arrangement also prevents the industrial hazards from entering the face of the wearer.

### BRIEF DESCRIPTION OF THE DRAWINGS

The above, and other objects, features and advantages of the present invention will become apparent from the detailed description read in conjunction with the accompanying drawings, which are given by way of illustration only, and thus are not limitative of the present invention, and wherein:
FIG. 1 is an exploded view illustrating one embodiment of an integrated mask and goggle structure of the present invention;
FIG. 2 is a partially cross sectional side view illustrating the device of FIG. 1 mounted on the face of a wearer;

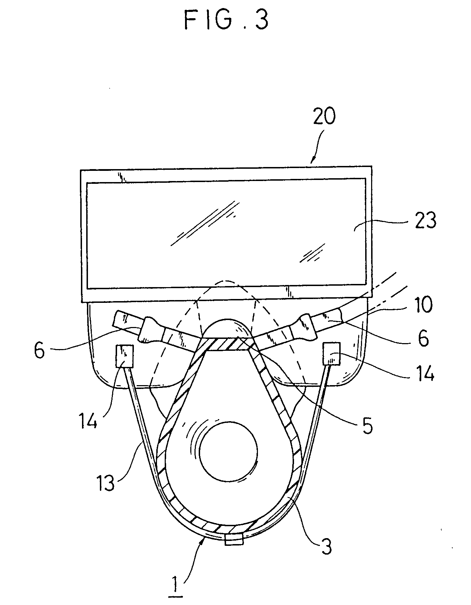

FIG. 3 is a partially cross sectional front view illustrating the device of FIG. 1 mounted on the face of the wearer;
FIGS. 4A and 4B are front views, each of which illustrating a mounting member of another embodiment of the device of the present invention;
FIG. 5 is a front view illustrating a mounting member of another embodiment of the device of the present invention;
FIG. 6A is a perspective view illustrating a mounting state of the device of FIG. 4A;
FIG. 6B is a partially enlarged cross sectional view taken along a line I-I;
FIG. 7 is an exploded view illustrating yet another embodiment of the device of the present invention;
FIG. 8 is a partially cross sectional side view illustrating the device of FIG. 7 mounted on the face of a wearer;
FIG. 9 is a front view illustrating the device of FIG. 7;
FIG. 10 is a fragmentary cross sectional view illustrating a mounting state between a goggle and a mounting member of the device of FIG. 7;
FIG. 11 is an exploded view illustrating another embodiment of the device of the present invention;
FIG. 12A is a side view illustrating the device of FIG. 11 mounted on the face of a wearer; and
FIG. 12B is a fragmentary cross sectional view illustrating an engagement state between a mounting member and a mask of FIG. 17;
FIG. 13A is a perspective view illustrating another embodiment of an integrated mask and goggle structure of the present invention;
FIG. 13B is a fragmentary cross sectional view illustrating a mounting state of the mask and the goggle of FIG. 13A;
FIG. 14 is a cross sectional view taken along a line II-II of FIG. 13A;
FIGS. 15A and 15B are side views illustrating the integrated mask and goggle structure of FIG. 13A respectively in a withdrawn position and an operative position on the face of a wearer;
FIG. 16 is a fragmentary cross sectional side view illustrating an embodiment of a sealing means between the mask and the goggle in the operative position of the device of FIG. 13A;
FIG. 17 is a fragmentary cross sectional side view illustrating another embodiment of the sealing means between the mask and the goggle;
FIG. 18 is a fragmentary cross sectional side view illustrating still another embodiment of the sealing means between the mask and the goggle;
FIGS. 19A and 19B are fragmentary cross sectional side views illustrating further embodiment of the sealing means;
FIG. 20 is a perspective view illustrating another embodiment of the device of the present invention;
FIG. 21A is a perspective view illustrating a guide member of another embodiment of the device of the present invention;
FIG. 21B is a cross sectional view taken along a line III-III of FIG. 21A;
FIG. 22A is a perspective view illustrating another embodiment of the present invention; and
FIG. 22B is a fragmentary cross sectional side view illustrating a connecting state between the mask and the goggle of FIG. 22A.

### DETAILED DESCRIPTION OF THE PREFERRED EMBODIMENTS

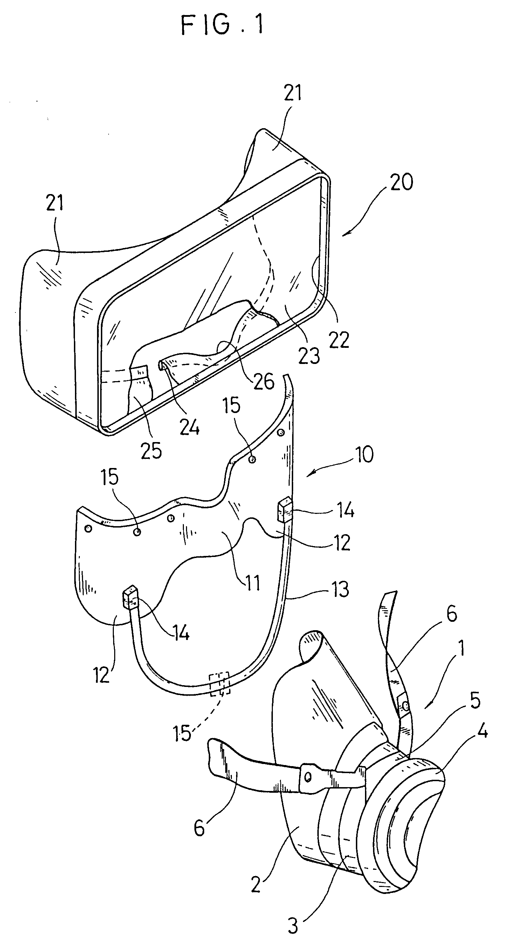

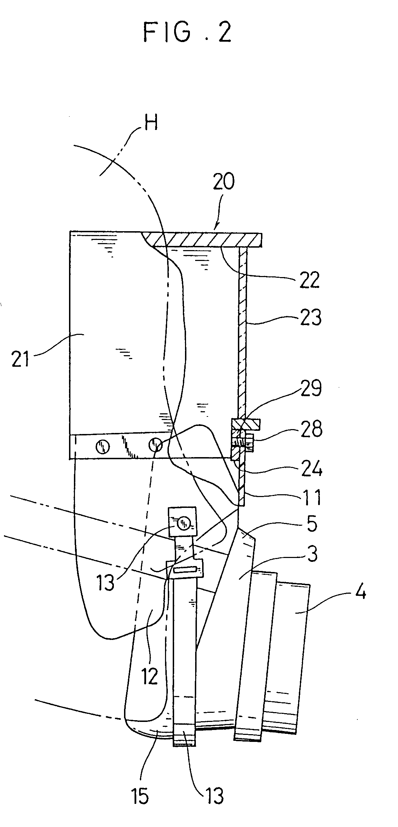

Referring to FIGS. 1 and 2 which illustrate one embodiment of the present invention, a goggle 20 made of metal or plastic is provided on its overall periphery with a protruding portion 21 curved to fit about the face of a wearer, defining an edge of the goggle. A lens 23 is secured to an opening 22 of the goggle 20. The lens 23 may be made of safety glass or plastic, clear or tinted. A fitting strip 24 is downwardly secured to the marginal edge of a lower strip 25 of the protruding portion 21 which carries a plurality of holes 29 with internal threads which are spaced apart from each other, and a nosepiece positioned at its center.

A mounting member 10 adapted to connect the goggle 20 to a mask 10 is formed of plastic and has a configuration such that it can fit to the fitting strip 24 of the goggle 20. On an upper end of the mounting member 10 is provided with a plurality of holes 15 which accomodate bolts 28 to be screwed into the holes 29 of the fitting strip 24 of the goggle 20. In the substantially center of the mounting member is provided a protruding portion which has a contour for fitting to the nosepiece 26 of the goggle 20. A pair of integral extensions 12 are provided on both sides of the mounting member 10, to which a flexible fastening belt 13 is secured via detent means 14. An attachment 15 is provided on a center of the fastening belt 13 to enable the wearer to detachably connect the attachment 15 and adjust the length thereof. Accordingly, the mounting member 10 can be securely mounted on the mask 1. The fastening belt 13 may be made from an elastic cord, spring member or the like.

The mask 1 comprises a mask body 3 of plastic with a flexible adjustable strap 6, a face contacting extension 2 made of elastic material which is secured to a rear side of the mask body 3, and a filter box 4 for filtering outside air which is secured to a front side of the mask body 3. The fastening belt 13 of the mounting member 10 is fastened around the mask body 3 such that the goggle 20, the mounting member 10 and the mask 1 can be integrated together. In this arrangement, the protruding portion 11 of the mounting member 10 and lower portions of the integral extensions 12 surround at least a marginal edge of the face contacting extension 2, and brought into engagement with an abutment surface 5 of the mask body 3 from above so as to prevent the goggle 20 from being accidentally slipped off from the mask 1 and prevent the dusts or the like from entering the inside of the face contacting extension 2.

When the device of the above arrangement is to be used, the mounting member 10 is first secured to the goggle via the bolts 28, then the lower portion of the mounting member is brought into engagement with the abutment surface 5 of the mask 1, and the goggle 20 is fastened to the mask 1 by means of the fastening belt 13 of the mounting member 10.

Then, the mask 10 and the goggle 20 respectively and snugly surround the mouth and the nose, and the eyes of the wearer by fastening the strap 6 of the mask 1 around the head H. Under this fitting state, the lower portion of the mounting member 10 is in tight contact with the face contacting extension 2 of the mask 1, whereby effectively preventing the intrusion of the dusts or the like into the mask 1.

Since the mounting member 10 is detachably mounted to the goggle 20, the mounting members 10 of various types are prepared in accordance with the mask 1 of different type, such as the mask 1 carrying two filter boxes 4. Whereby, the mask 1 can be interchanged for different purposes. In addition, since the goggle 20 and the mask 1 are integrally connected together merely via the fastening belt 13 of the mounting member 10, it is not necessary to apply any modifications to the mask 1, thus securing the broad range of the application thereof and reducing the manufacturing costs.

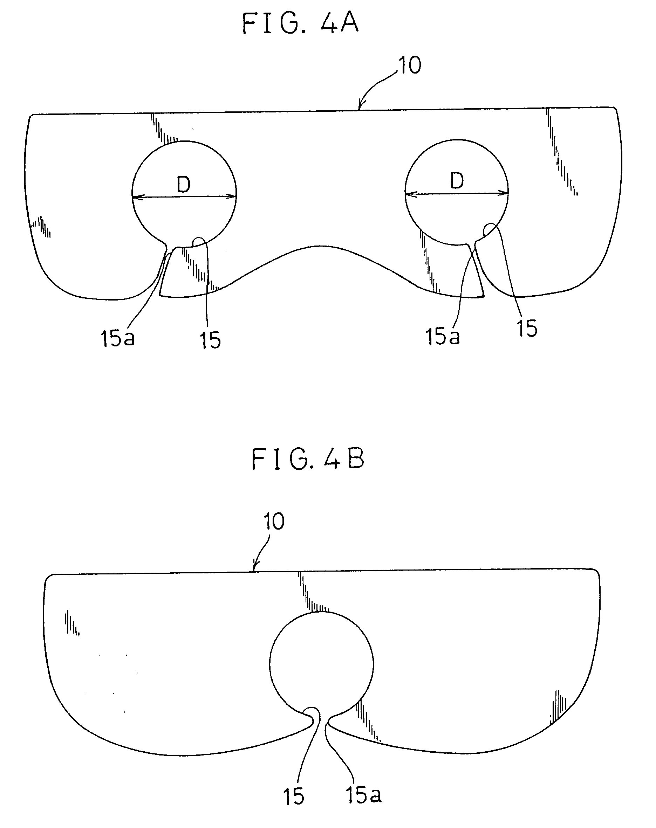

The second embodiment is provided for substituting the fastening belt 13 of the first embodiment as a means for fastening the mask 1 to the goggle 20 by a cut-out 15. In FIG. 4A, the mounting member 10 is made of a thin elastic sheet material made of for example, a soft plastic. A pair of the cut-outs 15 are formed in the mounting member 10 so as to fit to the mask 1 having a pair of the filter boxes 4. Each of the cut-outs 15 has a diameter D which is as large as or slightly larger than the outer diameter of the corresponding filter box 4. A slit 15a is formed between each of the cut-outs 15 and an outer periphery of the mounting member 10. When the mounting member 10 of this arrangement is to be secured to the mask 1, it is twisted around the slit 15a so as to expand the slit 15a and fit around the filter box 4 therethrough. Once the mounting member 10 is fitted on the mask 1, it comes back to the original shape due to its restoring force, thus securely holding the mask 1.

When the mask 1 having a single number of the filter box 4 is to be used, the cut-out 15 is formed at the substantially center of the mask 1 as illustrated in FIG. 4B.

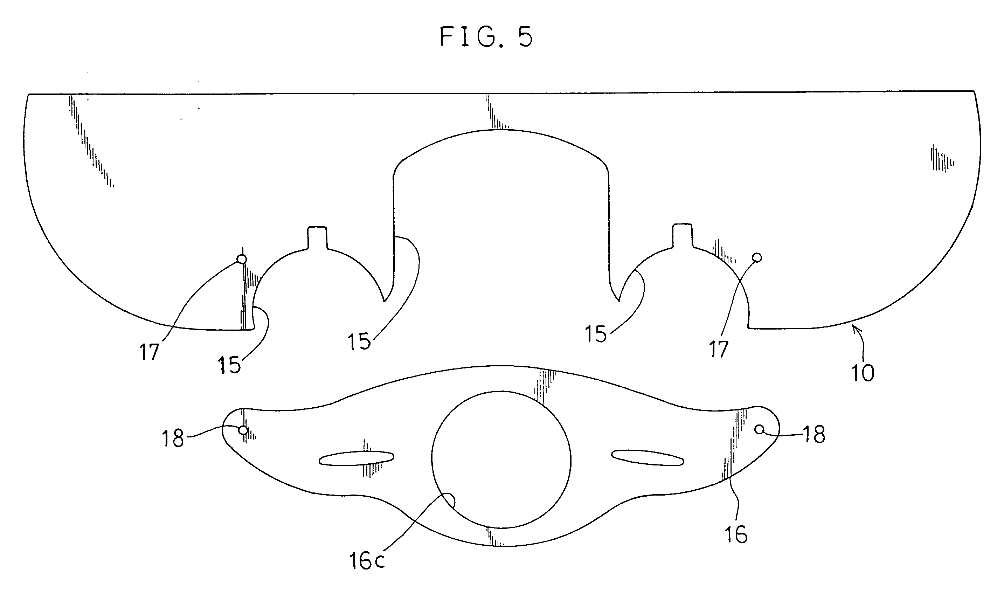

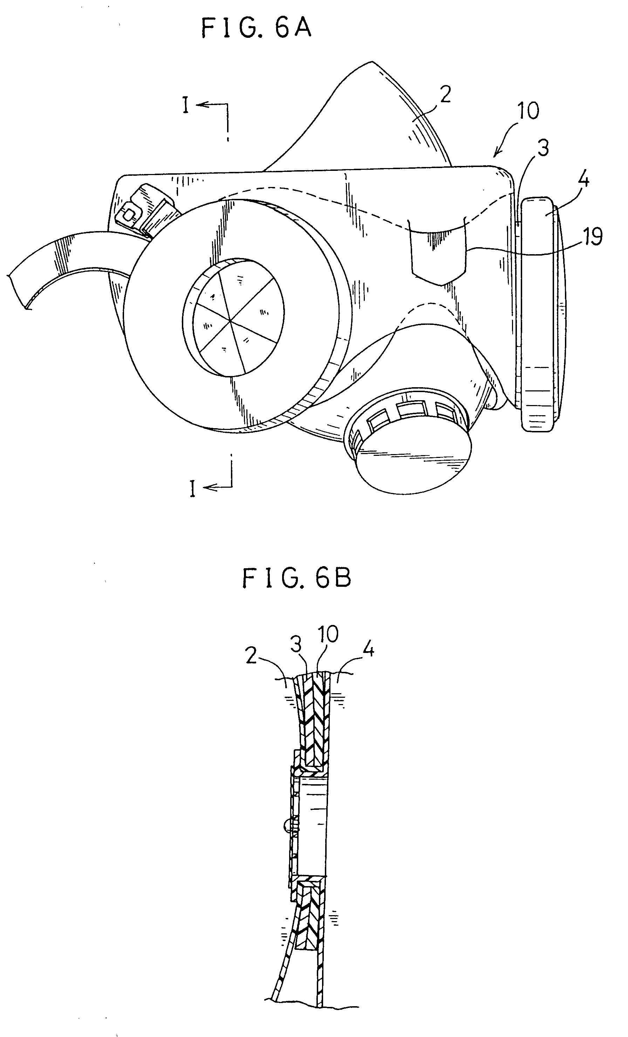

When the mask 1 has such a configuration that the cut-out 15 hardly secures the mask 1 in position, for example, a configuration in which the overall region of the rear side of the filter box 4 is attached on the mask body 1 which does not allow the insertion of the mounting member 10 therebetween, a fitting strip 16 is fitted on the mask from below and connected to the mounting member 10 with any conventional connecting means (not shown) inserted through holes 17 of the mounting member 10 and holes 18 of the fitting strip 18. The cut-outs 15 is of a half circle so as to fit on the filter box 4 from above. The three cut-outs 15 are arranged in the mounting member 10 as illustrated in FIG. 5 so as to accomodate the mask 1 having less than three filter boxes 4. In addition, to accomodate the mask 1 having an exhaust port, a cut-out 16c is preferably formed in the fitting strip 16. Referring to FIGS. 6A and 6B, the mounting member 10 is actually fitted on the mask 1, in which a tongue 19 defined by cutting a predetermined region of the mounting member 10 is preferably inserted between the mask body 3 and the face contacting extension 2 so as to prevent the outwardly bending of the mounting member 10. However, the tongue 19 is not an essential feature of the present invention. It is essential that the mounting member 10 is detachably mounted on the mask 1.

With this arrangement, it is not necessary to form the mounting member 10 in three dimensions which is required in the first embodiment. Merely the two dimensional mounting member is sufficient to attain the advantage as in the first embodiment, which may considerably reduce the manufacturing cost thereof. In addition, the thin plastic material employed for the mounting member 10 allows the cut-out 15 to be readily cut out from the mounting member 10 having merely a specific outline by the wearer or the like. Thus, the mounting member 10 of this type may be applied to the mask of varying configuration.

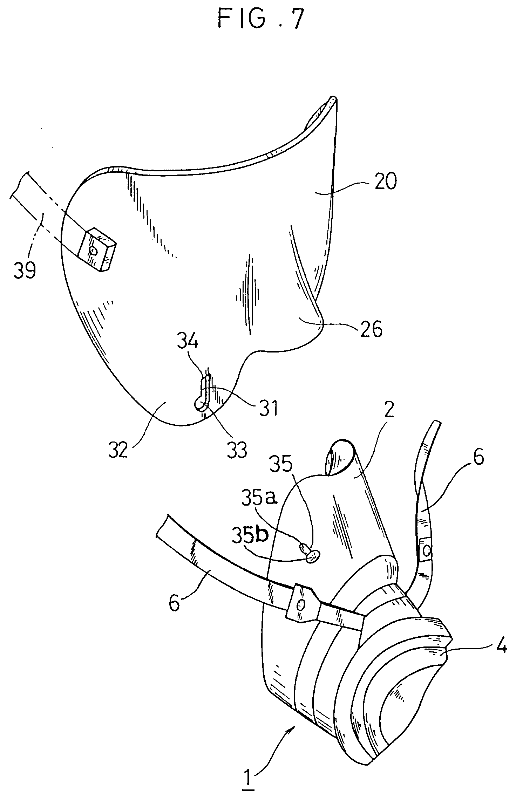

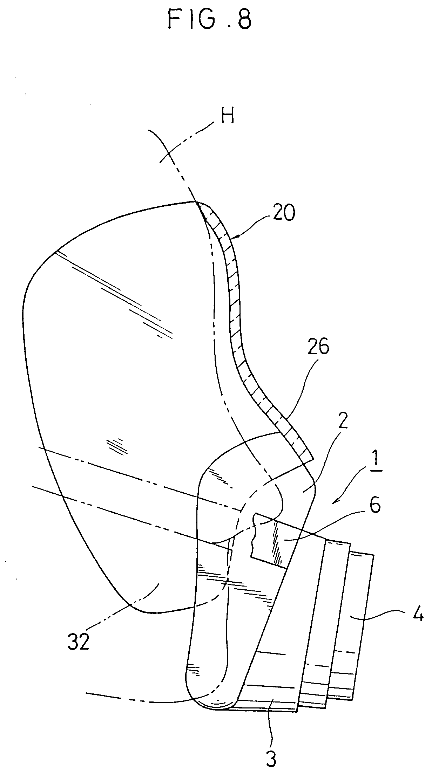

The third embodiment is illustrated in FIGS. 7, 8, 9 and 10. The goggle 20 is integrally formed of a transparent and hard plastic material and shaped so as to conform about the face of the wearer, in which the nosepiece 26 protrudes away from the substantially center of the goggle 20. On a lower portion of the goggle 20 is provided a pair of integral extensions 32, each of which carrying a detent opening 31 which in turn comprises a circular opening 33 and an elongated opening 34 which extends from the circular opening 33 and has a width smaller than the diameter of the circular opening 33.

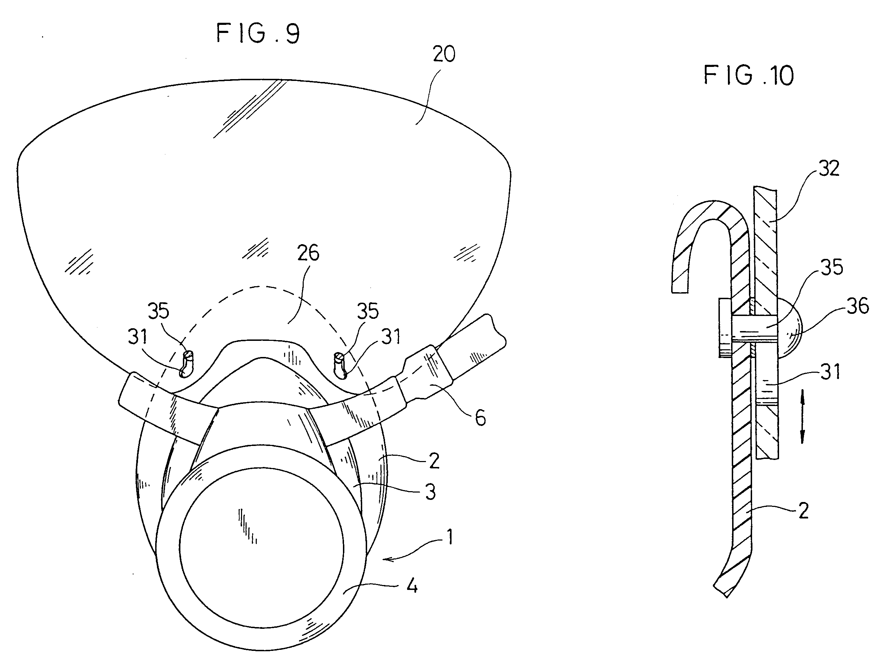

A pair of engagement protrusions 35 protrude forwardly away from the face contacting extension 2 of the mask 1, each of which carries a shank 35a secured to the face contacting extension 2, and a knob 35b at its tip end. The knob 35b has a diameter larger than the width of the elongated opening 34, but smaller than the circular opening 33. Each of the engagement protrusions 35 is inserted into the corresponding elongated opening 34 via the circular opening 33. The shank 35a of the engagement protrusion 35 travels within the elongated opening 34 as best illustrated in FIG. 10 so as to be held with the elongated opening 34, in which the knob 35b arrests the slipping off of the engagement protrusion 35 from the elongated opening 34. Accordingly the mask 1 is detachably connected to the goggle 20 via a fitting means, namely the detent hole 31 in the goggle 20 and the engagement protrusion 35 on the mask 1. After the mask 1 is secured to the goggle 20, the strap 6 is fastened around the head of the wearer as illustrated in the first embodiment so as to wear the integrated mask and goggle structure of this arrangement.

When it is desirable to detach the mask 1 from the goggle 20, the engagement protrusion 35 within the elongated opening 34 of the detent hole 34 is brought into the circular opening 33, then pulling out the engagement protrusion 35 therefrom. After the mask 1 is released from the goggle 20, the goggle 20 may be independently used by fastening a strap 39 which may be detachably fixed on the goggle 20.

Contrary to this arrangement, it is possible to provide the detent opening 31 in the mask 1 and the engagement protrusion 35 on the goggle 20.

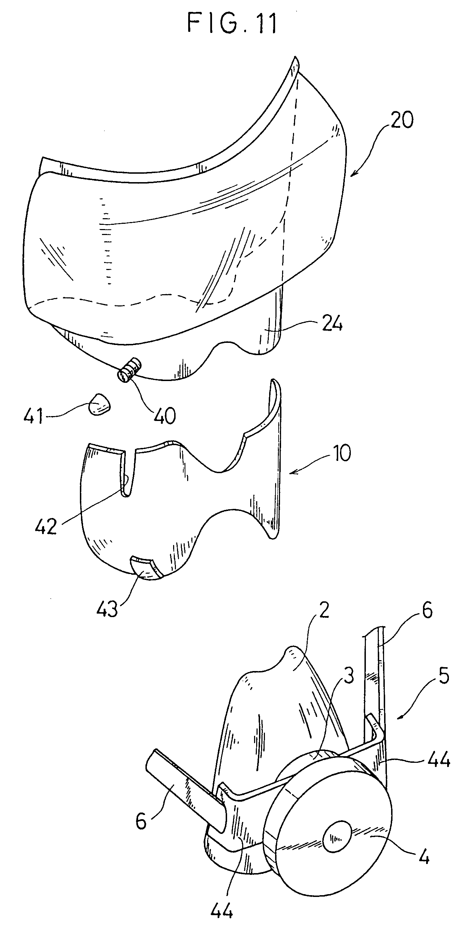

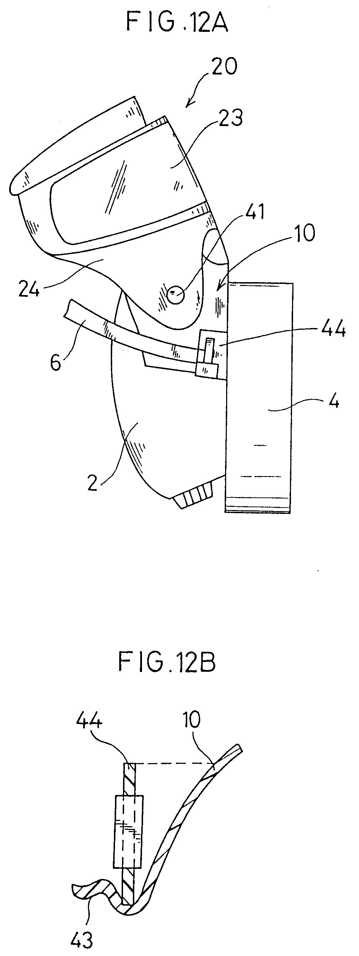

Referring to FIGS. 11, 12A and 12B illustrating the fourth embodiment, a pair of bolts 40 protrude forwardly away from the fitting strip 24 extending downwardly from the goggle 20 so as to be inserted into corresponding elongated openings 43 defined by an upper portion of the mounting member 10. The elongated opening 42 enables the mounting member 10 to be moved towards and away from the goggle 20 so as to adjust the position of the mounting member 10 to the goggle 20. Nuts 41 respectively accomodate the corresponding bolts 40.

A pair of connecting plates 44 respectively protrude away from the both sides of the mask body 3, to each end of which the strap 6 is fixed. On a lower portion of the mounting member 10 is provided a pair of upwardly orienting engagement strips 43 which are respectively brought into engagement with the connecting plates 44 from below, as illustrated in FIG. 12B. However, the upwardly orienting engagement strips 43 can be freely released from the engagement with the connecting plates 44, when desired.

In this embodiment, the number, configuration and dimension of the mounting means may also be varied depending on the goggle 20 and the mask 10 to be mounted. Thus, the goggle 20 and the mask 1 may be interchangeable in accordance with a different purpose.

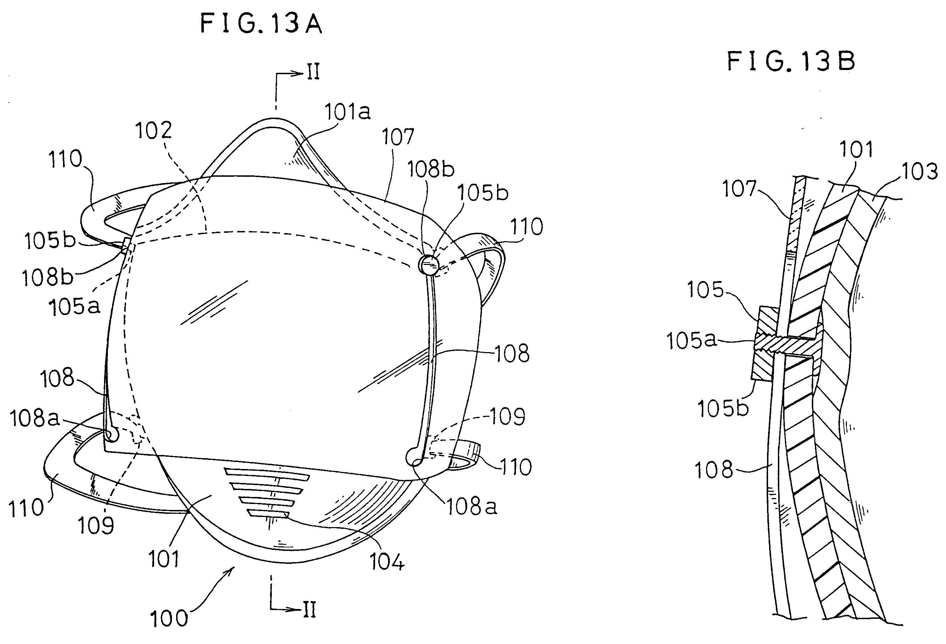

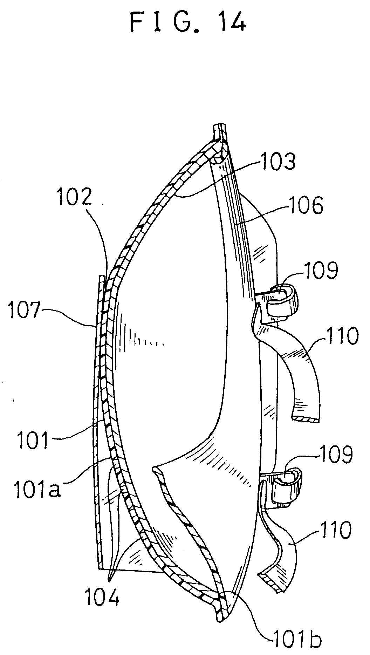

The forth embodiment is provided to illustrate another aspect of the present invention. In FIGS. 13A and 13B, a mask 100 has a mask body 101 which is of a semi-oval shape in cross section, and made of a hard plastic material. On an upper portion of a front surface 101a of the mask body 101 is provided with a bulge 102 as best illustated in FIG. 14. A filter layer 3 made of non woven fabric is secured to the reverse surface 101b of the mask body 101 by any suitable means. A plurality of slits 104 for ventilation are provided on a lower portion of the mask body 101. A pair of engagement protrusions 105 comprises a shank 105a with external threades, and a knob 105b with a threaded hole adapted to be threaded into the shank 105a as best illustrated in FIG. 13B. A flap 106 made of a soft plastic extends inwardly from a periphery of the reverse surface 101b of the mask body 101 so as to seal the perimeter of the mask 100 to the face of the wearer.

A goggle 107 is integrally made of a transparent and hard plastic material. In right and left hand sides of the goggle 107 are provided a pair of vertically extending slots 108 within which the corresponding shanks 105a of the mask body 101 travel. Each of the slots 108 carries a lower detent opening 108a and an upper detent opening 108b respectively in lower and upper ends thereof, where the vertical movement of the shanks 105a are arrested. The diameters of the detent openings 108a and 108b are larger than the width of the slot 108 such that the detent holes 108a and 108b project from the slot 108 towards the center of the goggle 107.

At upper and lower portions of right and left hand sides of the mask 101 are provided strap fastening means 109 to which flexible straps 110 made of rubber or the like are fastened.

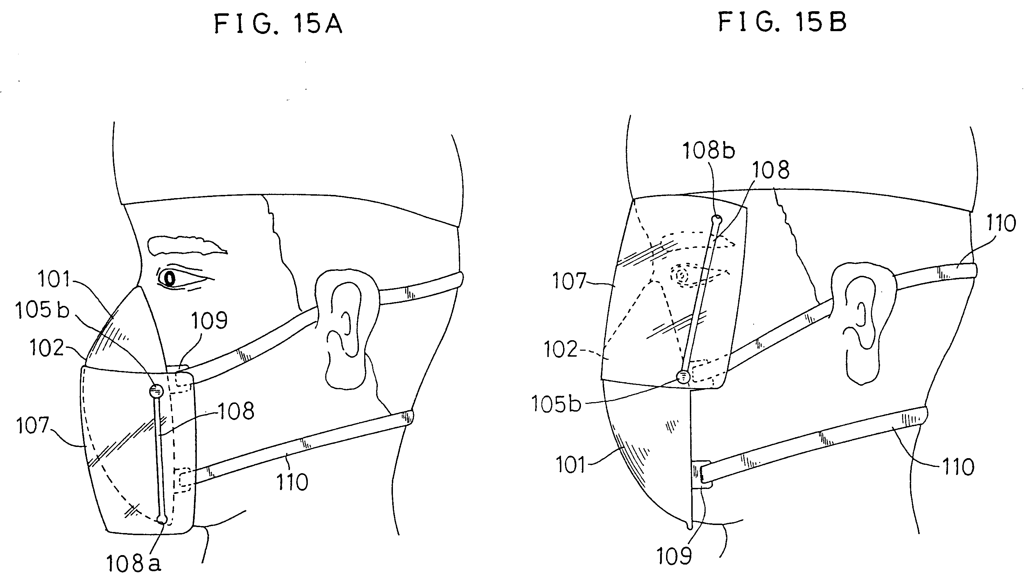

When the integrated mask and goggle structure of this arrangement is to be used for welding, surgecal operation or the like, the goggle 107 is first mounted on the mask 100 by inserting the shanks 105a of the mask body 101 into the slots 108, and screwing the knobs 105b into the shanks 105a. The goggle 107 is preferably positioned in a withdrawn position where the shanks 105a are positioned in the upper detent openings 108b, for the easy mounting on the face, as illustrated in FIG. 15A. Since the goggle 107 of a plastic material is forced to be slightly curved so as to conform about the mask 100 when it is mounted on the mask 101, the centrally projecting portions of the detent openings 108a and 108b spontaneously move towards the shanks 105b when they are at the ends of the slots 108. Whereby, the goggle 107 is securely held in the withdrawn position. The integrated mask 101 and the goggle 107 are mounted on the face of the wearer by fastening the straps 110 around the head of the wearer.

When the goggle 107 is to be moved upwardly so as to surround the face, the goggle 107 is first bent by pushing its opposite vertical sides inwardly so as to release the engagement between the shanks 105a and the upper detent openings 108b. Then, the shanks 105a travel within the slots 108 and come into engagement with the lower detent openings 108a, where the goggle 107 is securely held in an operative position where the goggle 107 sufficiently surrounds the eyes of the wearer. The bulge 102 of the mask body 101 seals between the mask 100 and a lower portion of the goggle 107, arresting the intrusion of the industrial hazards such as dusts and the poisonous gases into the face of the wearer at the time of the the operative position of the goggle 107.

Since the mask 101 and the goggle 107 are integrated in accordance with the above arrangement, they can be simultaneously worn, thus omitting the troublesome work which may be required for individually wearing the mask and the goggle.

With this arrangement, when it is desirable to expose the face of the wearer, for example, for the purpose of wiping the perspiration from the forehead in a hot or humid climate, it is easy to slide the goggle 107 downwardly along the slots 105, while wearing the mask 100 on the face of the wearer, thus contributing to an effective work of the wearer.

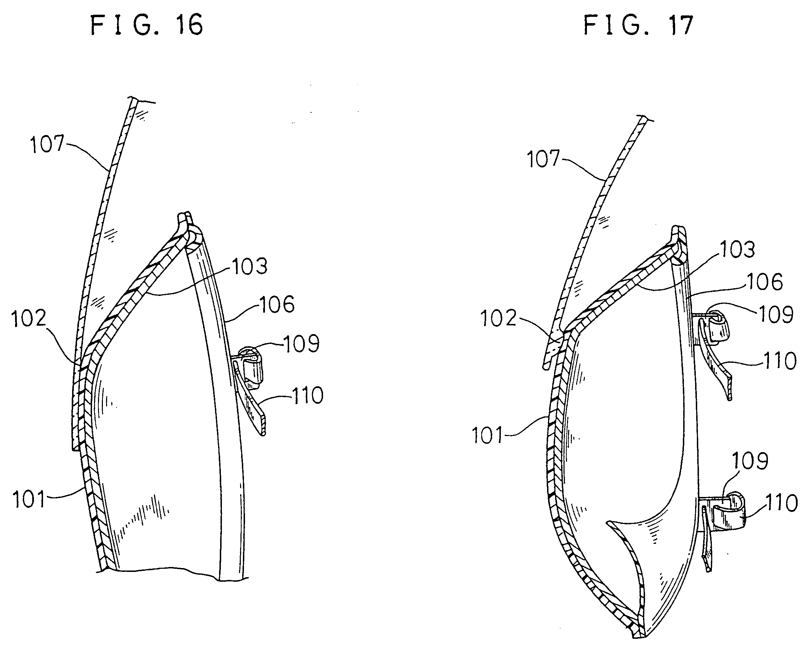

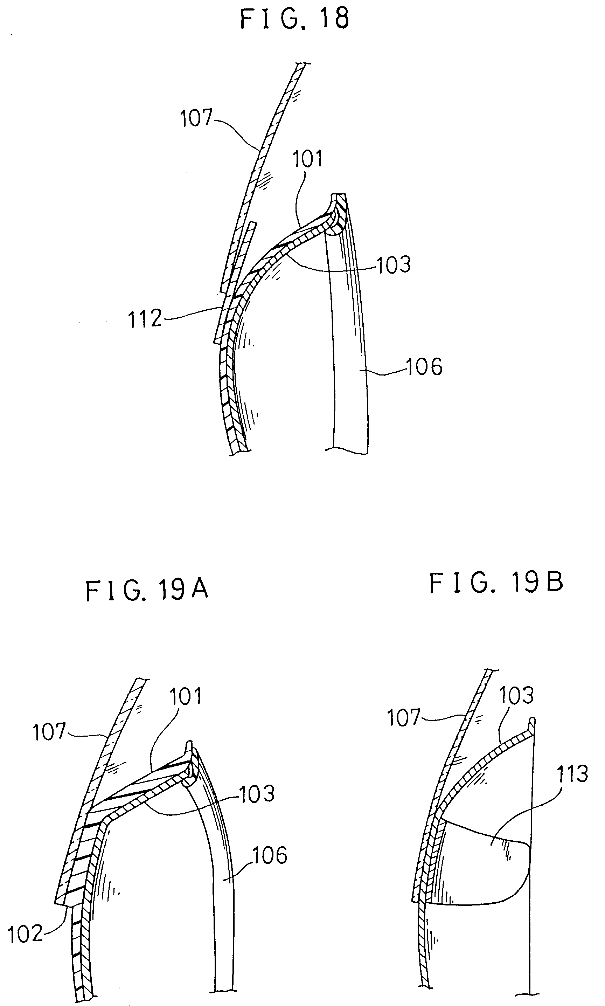

In this arrangement, the bulge 102 is integrally formed on the mask 101 to seal between the mask 101 and the goggle 107. However, the bulge 102 may be provided on the reverse surface of the lower portion of the goggle 107 as illustrated in FIG. 17. Further, as illustrated in FIG. 18, a sealing strip 112 of an elastic material may be secured to the front surface 101a of the mask body 101 so as to tightly abut against the reverse surface of the goggle 107, in which the strip 112 elastically and forcibly push the goggle 107, thus ommitting a clearance between the mask body 101 and the goggle 107. In this regard, the sealing strip 112 may be provided on the reverse surface of the goggle 107. Referring to FIG. 19A, the bulge 102 is formed by a stepped portion on the mask body 101 against which the goggle 107 abuts.

When the mask body 101 is not used in this arrangement, the filter layer of non woven fabric may form the mask, and a reinforcing strip 113 pushes the filter layer 3 outwardly so as to shape the bulge 102, as illustrated in FIG. 19B.

As apparent from the above description, a means for sealing between the mask 101 and the goggle 107 may be varied provided that the sealing condition is sufficiently maintained.

Instead of the slot 108 in the goggle 120, grooved strips (not shown) may be attached to the reverse surface of the goggle 120 so as to accomodate the engagement protrusions 105 of the mask 101. Accordingly, it is not necessary to define any opening in the goggle 107. Whereby, the industrial hazards which may enter the face via the opening of the goggle 107 can be effectively avoided.

In the above arrangement, the filter layer 103 is secured to the mask body 101. However, the filter layer 103 may be detachably provided on the mask body 101 via any suitable detent means so as to be interchanged, if desired.

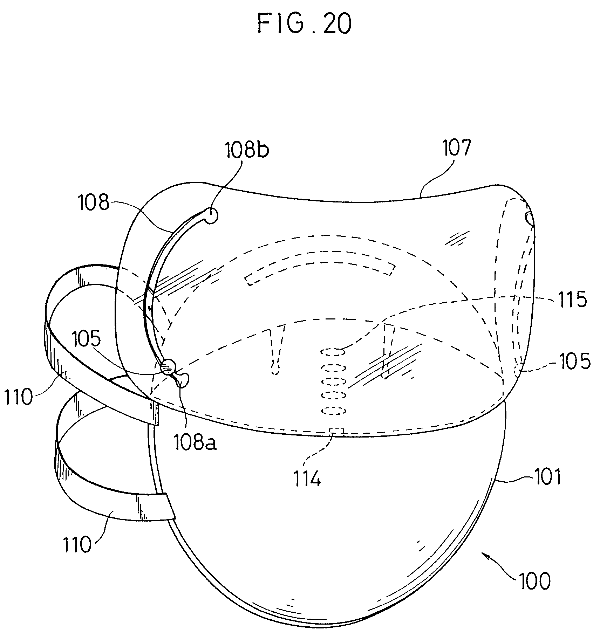

Further, instead of the vertically extending slot 108 of the above embodiment, the slot 108 may be of a circular arc when the goggle is made of elastic synthetic resin, as illustrated in FIG. 20. With this arrangement, the goggle 107 may readily be moved between the operative position and the withdrawn position by reducing the outwardly expanding force of the goggle 107 exerted by the restoration force of the goggle 107.

Referring to FIG. 20, in addition to the detent openings 108a and 108b respectively defined in the upper and lower ends of the slot 108, an integral stud 114 is provided on the lower edge of the goggle, protruding inwardly so as to be brought into engagement with a plurality of cut-outs 115 provided on the mask body 101. With this arrangement, the goggle 107 can be held at various heights, which may contribute to the efficient work of the wearer.

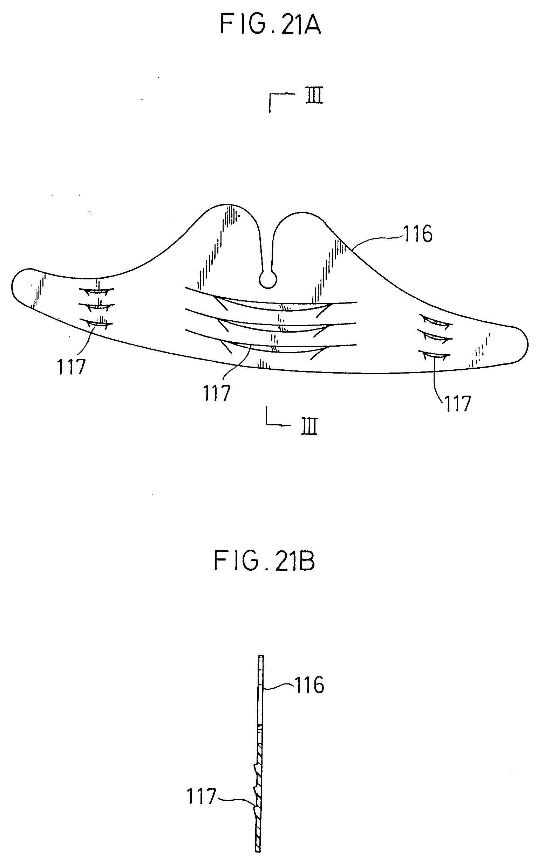

FIGS. 21A and 21B illustrate a guide member 116 which is made of plastic. Three stages of stepped portions 117 are respectively formed on the center and opposite sides of the guide member 116. The guide member 116 is attached on the front surface 101a of the mask body 101 via an adhesive or other suitable means so as to receive the lower edge of the goggle 107 and hold the goggle 107 at various heights. With this arrangement, the integral stud 114 and the cut-out 115 of FIG. 20 may be omitted.

As apparent from the above description, it is not necessary to limit the detent means for holding the goggle 107 in position to the above embodiment. Various modifications may be made provided that it can hold the goggle 107 at a predetermined height.

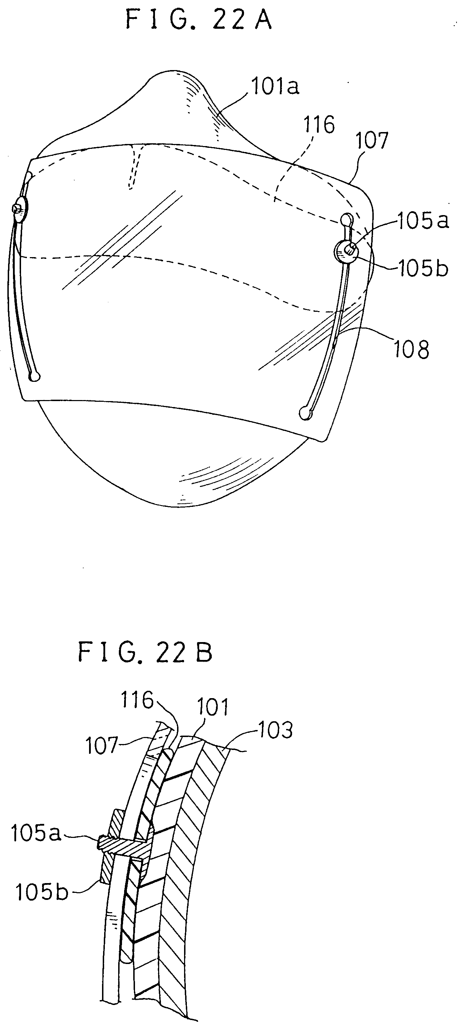

Referring to FIGS. 22A and 22B illustrating a further embodiment, a mounting member 120 is attached on the mask body 101 by any suitable means such as an adhesive (not shown). A pair of the engagement protrusions 105 protrude forwardly away from the mounting member 120 so as to be respectively brought into engagement with the slots 108 of the goggle 107. Whereby, the goggle 107 is slidably mounted on the mask 101 via the mounting member 120.

In the above arrangement, the engagement protrusion 105 comprises the shank 105a and the knob 105b such that the goggle 107 can be detachably mounted on the mask 101. However, when it is not necessary to interchange the mask 101 and the goggle 107, the engagement protrusion 105 may be unitarily made. With this arrangement, the goggle 107 is undetachably mounted on the mask 101.

This specification is by no means intended to restrict the present invention to the preferred embodiments set forth therein. Various modifications to the inventive integrated mask and goggle structure as described herein, may be made by those skilled in the art without departing from the spirit and scope of the present invention.

## Claims

1. An integrated mask and goggle structure adapted to be mounted on the face of a wearer and protect the face of the wearer against industrial hazards or the like comprising a mask (1) adapted to surround the mouth and the nose of a wearer, and a goggle (20) adapted to surround the eyes and detachably mounted to the goggle.

2. The integrated mask and goggle structure as set forth in claim 1, wherein the structure further comprises a mounting member (10) adapted to be fitted to a lower portion of the goggle (20), and a mounting means provided on the mounting member so as to detachably mount the mask thereto.

3. The integrated mask and goggle structure as set forth in claim 2, wherein the mounting means comprises a flexible cord (13) adapted to be fastened around the mask (1).

4. The integrated mask and goggle structure as set forth in claim 2, wherein the mounting means is a detent opening defined in either the mounting member (10) or the mask (1), and an engagement protrusion provided on the other so as to be brought into engagement with the detent opening.

5. The integrated mask and goggle structure as set forth in claim 2, wherein the mask (1) comprises a mask body (3), a face contacting extension (2) provided on a rear side of the mask body (3) and a filter box (4) provided on a front side of the mask body (3), and the mounting means comprises a cut-out (15) defined in the mounting member (10) having a configuration adapted to surround the mask body (3).

6. An integrated mask and goggle structure adapted to be mounted on the face of a wearer and protect the face of the wearer against industrial hazards or the like comprising a mask (1) adapted to surround the mouth and the nose of a wearer, and a goggle (20) adapted to surround the eyes, and a mounting means disposed between the mask (1) and the goggle (20) such that the goggle (20) is detachably mounted to the mask (1).

7. The integrated mask and goggle structure as set forth in claim 6, wherein the mounting means comprises a detent opening (31) and an engagement protrusion (35) adapted to be brought into engagement with the detent opening (31).

8. The integrated mask and goggle structure as set forth in claim 1, wherein the structure further comprises a sliding means for vertically sliding the goggle mounted to the mask between its operative position and withdrawn position.

9. An integrated mask and goggle structure adapted to be mounted on the face of a wearer and protect the face of the wearer against industrial hazards or the like comprising a mask (100) adapted to surround the mouth and the nose of the wearer, and a goggle (107) adapted to surround the eyes, wherein the goggle (107) is slidably mounted to the mask (100) so as to be moved between its operative position and withdrawn position.

10. The integrated mask and goggle structure as set forth in claim 9, a slot (108) provided on the goggle (107), and an engagement protrusion (105) provided on the mask (100), wherein the engagement protrusion (105) is brought into engagement with the slot (108) such that the goggle (107) is securely moved between its operative position and withdrawn position.

11. The integrated mask and goggle structure as set forth in claim 10, wherein the engagement protrusion (105) comprises a shank (105a) of a diameter smaller than the width of the slot (108) and a knob (105b) of a diameter larger than the width of the slot (108) provided at its tip end, a pair of the slots (108) respectively and vertically extend along the right and left hand sides of the goggle (107) and terminate at detent openings (108a, 108b) adapted to arrest the vertical movement of the engagement protrusions (105) of the mask (100) such that the goggle (107) is securely held in its operative position and withdrawn position.

12. The integrated mask and goggle structure as set forth in claim 9, wherein the mask (100) comprises a mask body (101), and a filter layer (103) of non woven fabric provided on a reverse surface (101b) of the mask body (101), and a sealing means provided between the mask (100) and the goggle (107) so as to seal therebetween when the goggle (107) is held in its operative position.

13. The integrated mask and goggle structure as set forth in claim 12, wherein the sealing means comprises a bulge (102) provided either on an upper portion of a front surface (101a) of the mask body or on a lower portion of a reverse surface of the goggle so as to be brought into tight contact with the adjacent surface when the goggle (107) is held in its operative position.

14. The integrated mask and goggle structure as set forth in claim 12, wherein the sealing means comprises a horizontally extending strip (112) provided either on a front surface (101a) of the mask body (101) or along the lower end of the goggle (107) so as to be brought into tight contact with the adjacent surface.

15. The integrated mask and goggle structure as set forth in claim 9, wherein the mask (100) comprises a filter layer (103) of non woven fabric, and a reinforcing strip (113) provided on the reverse surface of the filter layer so as to shape a bulging portion of the filter layer and seal between the mask (100) and the goggle (107) therewith when the goggle (107) is held in its operative position.

16. The integrated mask and goggle structure as set forth in claim 9, wherein the mask (100) comprises a mask body (101), and a filter layer (103) of non woven fabric detachably provided on the reverse surface (101b) of the mask body.

17. The integrated mask and goggle structure as set forth in claim 9, wherein the mask (100) comprises a mask body (101), a filter layer (103) made of non woven fabric provided on the reverse surface (101b) of the mask body, and an inwardly extending flexible flap (112) provided along a periphery of the reverse surface of the mask body (101) so as to seal the perimeter of the mask (100) to the face of the wearer.

18. An integrated mask and goggle structure adapted to be mounted on the face of a wearer and protect the face of the wearer against industrial hazards or the like comprising a mask (100) adapted to surround the mouth and the nose of a wearer, and a goggle (107) adapted to surround the eyes, a mounting member (116) adapted to be fitted to a lower portion of the goggle (107), a means (105, 108) for slidingly mounting the goggle (107) to the mask (100) provided on the mounting member, wherein the goggle (107) is slidably mounted to the mask (100) so as to be moved between its operative position and withdrawn position.
